# EUROPEAN PATENT APPLICATION

(11) **EP 4 585 206 A1**
(43) Date of publication of application: **16.07.2025**
(21) Application number: 23880158.3
(22) Date of filing: 17.10.2023
(51) Int. Cl.: A61K 9/00, A61K 31/05, A61P 19/02, A61P 29/00

(54) **SUSTAINED-RELEASE INJECTABLE COMPOSITION FOR TREATING OR PREVENTING INFLAMMATORY DISEASE, AND METHOD FOR PREPARING SAME**

(30) Priority: 17.10.2022 KR 20220133524
(71) Applicant: Inventage Lab Inc., Gyeonggi-do 13403 (KR); Yuhan Care Co., Ltd., Seoul 07789 (KR)
(72) Inventor: KIM, Ju Hee, Seongnam-si, Gyeonggi-do 13494 (KR); SEO, Young Dai, Seongnam-si, Gyeonggi-do 13494 (KR); PARK, Hyun Je, Gunpo-si, Gyeonggi-do 15889 (KR); CHA, Joo Young, Yongin-si, Gyeonggi-do 16944 (KR); SONG, Aeri, Hwaseong-si, Gyeonggi-do 18441 (KR)
(74) Representative: BCKIP Part mbB
(86) International application number: PCT/KR2023/015992
(87) International publication number: WO 2024/085581

(57) **Abstract**

The present invention relates to a sustained-release injectable composition for treating or preventing an inflammatory disease, and a method for preparing the same. The present invention may exhibit the effect of treating or preventing inflammatory diseases by releasing cannabidiol in a sustained manner for one month or more using microparticles containing cannabidiol. **In** addition, the sustained-release injectable composition containing cannabidiol is not toxic, may be used continuously for a long period of time without side effects caused by long-term use, and may exhibit a therapeutic or preventive effect on inflammatory diseases for one month or more by a single injection, thereby significantly improving dosing convenience.

## Description

### Technical Field

The present invention relates to a sustained-release injectable composition for treating or preventing inflammatory diseases and a method for preparing the same.

### Background Art

Osteoarthritis is a degenerative disease caused by damage to the knee cartilage and abnormalities in the synovial fluid, and refers to arthritis caused by degenerative changes in the cartilage and adjacent bone in the synovial joints. Cartilage is a slippery tissue that covers the ends of bones in joints, and healthy cartilage helps absorb shock generated when bones move against each other. In osteoarthritis, the upper layer of cartilage breaks down and wears away, which allows the bones under the cartilage to rub together. This causes pain, swelling, and loss of motion of the joint, and can cause the joint to lose its normal shape over time, leading to osteophytosis. In addition, pieces of bone or cartilage can break off and float within the joint space, causing further pain and damage.

People with osteoarthritis often experience joint pain and reduced movement. Unlike other forms of arthritis, osteoarthritis affects only joints, not internal organs. Osteoarthritis is the most common form of arthritis, and the second most common form of arthritis is rheumatoid arthritis, which is caused by an autoimmune disorder.

It is currently known that drug therapy, exercise therapy, or surgical therapy may be used to treat osteoarthritis. As drug therapy among them, drugs such as acetaminophen, nonsteroidal anti-inflammatory drugs (NSAIDs), and corticosteroids are used.

However, most drugs only treat inflammation and relieve pain, but do not fundamentally suppress cartilage degeneration. Rather, these drugs accelerate cartilage damage or have side effects on the cardiovascular system, gastrointestinal tract, kidney, liver, and the like.

Accordingly, there is a need to develop a therapeutic agent for treating or alleviating osteoarthritis, and there is a need to develop a product that may exhibit an increased effect of treating or preventing osteoarthritis through long-term sustained drug release by a single injection.

### [Prior Art Documents]

### [Patent Documents]

(Patent Document 1) KR 10-2020-0066281 A1

### DISCLOSURE

### Technical Problem

An object of the present invention is to provide a sustained-release injectable composition for treating or preventing inflammatory diseases and a method for preparing the same.

Another object of the present invention is to provide a sustained-release injectable composition that may exhibit an effect of treating or preventing inflammatory diseases by releasing cannabidiol in a sustained manner for one month or more using microparticles containing cannabidiol.

Still another object of the present invention is to provide a method for preparing a sustained-release injectable composition containing cannabidiol, which is non-toxic, has no side effects caused by long-term use, may be used continuously for a long period of time, and may exhibit the effect of treating or preventing inflammatory diseases for one month or more by a single injection, thereby significantly improving dosing convenience.

### Technical Solution

To achieve the above objects, the present invention provides a sustained-release injectable composition for treating or preventing an inflammatory disease, comprising microparticles that release cannabidiol (CBD) in a sustained manner for one month or more, wherein the microparticles may comprise cannabidiol and a biodegradable polymer.

The microparticles may comprise cannabidiol and the biodegradable polymer at a weight ratio of 1:3 to 1:10.

The biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

The polylactide-co-glycolide may have a monomer ratio of lactide: glycolide of 60: 40 to 90: 10.

The inflammatory disease may be osteoarthritis.

The microparticles may release 35% to 80% of the total weight of CBD, as measured at 18 hours in a release test conducted under the following release test conditions:

### [Release test conditions]

A dissolution test solution is prepared by adding Tween 20 to 1 L of 10 mM PBS buffer (pH 7.4) to 0.05% (w/v), thus preparing a mixed solution, and adding sodium azide and sodium ascorbate to the mixed solution to 0.02% (w/v) and 0.0625% (w/v), respectively.

100 mL of the dissolution test solution is placed in a vial and the microparticles are added thereto in such an amount that the total amount of CBD is 15 mg. After completely sealing the vial, the test is conducted by shaking the vial at 120 rpm while maintaining 50°C. At 18 hours after the start of dissolution, the vial is taken out and left for 3 minutes. Then, 1 mL of the test solution is accurately taken using a syringe, and centrifuged at 3,000 rpm for 3 minutes, and the supernatant is used as a test solution to measure the release of CBD.

A method for preparing a sustained-release injectable composition for treating or alleviating an inflammatory disease according to another embodiment of the present invention may include steps of: preparing an oil phase solution by dissolving cannabidiol (CBD) and a biodegradable polymer in an organic solvent; preparing an aqueous phase solution by dissolving a surfactant in water; and mixing the oil phase solution and the aqueous phase solution to form an emulsion.

The method may further include steps of: collecting the formed emulsion in the aqueous phase solution to remove residual solvent; washing and freeze-drying the emulsion from which the residual solvent has been removed, thereby producing microparticles; and mixing the freeze-dried microparticles with water for injection.

The oil phase solution and the aqueous phase solution may be injected into the respective microchannels and allowed to flow therethrough, and the emulsion including cannabidiol and the biodegradable polymer may be formed at a point where the flow of the oil phase solution and the flow of the aqueous phase solution intersect each other.

The oil phase solution may further contain butylated hydroxytoluene (BHT).

### Advantageous Effects

The present invention may exhibit the effect of treating or preventing inflammatory diseases by releasing cannabidiol in a sustained manner for one month or more using microparticles containing cannabidiol.

In addition, the composition of the present invention is a sustained-release injectable composition containing cannabidiol, which is known to have a wide margin of safety upon systemic exposure, and it has fewer side effects caused by long-term use and may exhibit a therapeutic or preventive effect on inflammatory diseases for one month or more by a single injection, thereby significantly improving dosing convenience.

### Brief Description of Drawings

FIG. 1 shows the results of a CBD release experiment for microparticles containing CBD according to one example of the present invention.
FIG. 2 shows the results of a rat pharmacokinetic experiment for microparticles containing CBD according to one example of the present invention.
FIG. 3 shows the results of measuring body weight changes in an osteoarthritis model administered microparticles containing CBD according to one example of the present invention.
FIG. 4 shows the results of measuring hind limb weight bearing in an osteoarthritis model administered microparticles containing CBD according to one example of the present invention
FIG. 5 shows the results of a paw withdrawal threshold test in an osteoarthritis model before administering microparticles containing CBD according to one example of the present invention.
FIG. 6 shows the results of a paw withdrawal threshold test in an osteoarthritis model 2 hours after administration of microparticles containing CBD according to one example of the present invention.
FIG. 7 shows the results of a paw withdrawal threshold test in an osteoarthritis model 4 days after administration of microparticles containing CBD according to one example of the present invention.
FIG. 8 shows the results of a paw withdrawal threshold test in an osteoarthritis model 7 days after administration of microparticles containing CBD according to one example of the present invention.
FIG. 9 shows the results of a paw withdrawal threshold test in an osteoarthritis model 11 days after administration of microparticles containing CBD according to one example of the present invention.
FIG. 10 shows the results of a paw withdrawal threshold test in an osteoarthritis model 14 days after administration of microparticles containing CBD according to one example of the present invention.
FIG. 11 shows the results of a paw withdrawal threshold test in an osteoarthritis model 21 days after administration of microparticles containing CBD according to one example of the present invention.
FIG. 12 shows the results of a paw withdrawal threshold test in an osteoarthritis model 28 days after administration of microparticles containing CBD according to one example of the present invention.
FIG. 13 shows the results of evaluating the stability of microparticles containing CBD according to one example of the present invention.

### Best Mode

The present invention relates to a sustained-release injectable composition for treating or preventing an inflammatory disease, comprising microparticles that release cannabidiol (CBD) in a sustained manner for one month or more, wherein the microparticles comprise cannabidiol and a biodegradable polymer.

### Mode for Invention

Hereinafter, embodiments of the present invention will be described in detail so that those skilled in the art can easily implement the present invention. However, the present invention may be embodied in various different forms and is not limited to the embodiments described herein.

Cannabidiol (CBD) that is used in the present invention is a physiologically active substance isolated from cannabis. It may be extracted directly from cannabis, or may be extracted CBD, or may be produced by chemical synthesis, and any salt thereof may also be used, without being limited to the above examples.

Cannabis is also called hemp, and was used as cloth in the past. More specifically, hemp fibers are used for fabrics, mosquito nets, ropes, fishing nets, and papermaking materials, and hemp fruits are squeezed to obtain oil, which is used for food, lamp oil, soap, varnish, and paint, and oil cake thereof is used as feed and fertilizer.

The psychoactive chemical delta-9-tetrahydrocannabinol (THC), one of the main components of cannabis, is a component derived from the *Cannabis sativa* or *Cannabis indica* plant, commonly called marijuana, and is classified as an illegal drug in some countries. In countries where it is classified as an illegal drug, storage and handling without special permission are considered as illegal acts. Another representative component is cannabidiol (CBD), which is known to have no psychoactive effects unlike THC. Therefore, various studies have been conducted recently on its use in medicines and cosmetics.

The World Health Organization (WHO) published a report stating that cannabidiol (CBD) oil based on a cannabis extract is effective against epilepsy, Alzheimer's disease (dementia), and the like.

The World Anti-Doping Agency has published the "2018 International Standard for Prohibited Lists" and excluded CBD from the list of prohibited drugs starting this year. CBD is a drug widely used by athletes for pain treatment. Other substances that may be obtained from cannabis, such as hashish and marijuana, have been banned, but CBD oil for medical purposes has been permitted.

As mentioned above, the use of CBD for medical purposes is permitted in various countries.

However, when CBD is used for medical purposes as mentioned above, it is used in the form of CBD oil. Thus, there is no formulation that increases the convenience of taking CBD, as CBD must be taken in a sustained manner for a long period of time to treat or prevent a specific disease.

Accordingly, the present invention provides an injectable composition that may be used for the treatment or prevention of inflammatory diseases using CBD, and is particularly characterized by comprising microparticles capable of releasing CBD in a sustained manner for one month or more by a single injection.

The microparticles are characterized by comprising CBD and a biodegradable polymer.

Accordingly, when the microparticles of the present invention are administered by injection, they may exhibit the effect of administering CBD in a sustained manner by a single administration. Specifically, the microparticles may maintain the blood concentration of CBD at the effective concentration or higher for one month or more by a single administration, may maintain the blood concentration of CBD at the effective concentration or higher for several months by a single administration, may maintain the blood concentration of CBD at the effective concentration or higher for three months by a single administration, may maintain the blood concentration of CBD at the effective concentration or higher for six months by a single administration, and may maintain the blood concentration of CBD at the effective concentration or higher for 12 months by a single administration.

That is, the microparticles may exhibit the effect of releasing CBD in a sustained manner for 1 month or more, 3 months or more, 6 months or more, or 12 months or more.

The effect of releasing CBD in a sustained manner as described above may be affected by the degradation rate of the sustained-release agent, i.e., the biodegradable polymer.

The biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

As an example, the molar ratio of glycolide to lactide in the polylactide-co-glycolide may be about 40:60 to about 90:10, about 40:60 to about 85:15, about 40:60 to about 80:20, about 40:60 to about 75:25, about 45:55 to about 90:10, about 45:55 to about 80:20, without being limited to the above examples. Preferably, the molar ratio of glycolide to lactide in the polylactide-co-glycolide may be about 75:25 or about 50:50. When a polylactide-co-glycolide having a molar ratio of glycolide to lactide within the above range is used as a sustained-release agent, it may release CBD *in vivo* in a sustained manner for one month or more.

The biodegradable polymer may include one or more types of polylactide and one or more types of polylactide-co-glycolide. In the present invention, the biodegradable polymer may include, for example, two types of polylactide, a combination of one type of polylactide and one type of polylactide-co-glycolide, two types of polylactide-co-glycolide, three types of polylactides, a combination of two types of polylactide and one type of polylactide-co-glycolide, a combination of one type of polylactide and two types of polylactide-co-glycolide, or the like, and in particular, may include a combination of one type of polylactide and one type of polylactide-co-glycolide, or two types of polylactide-co-glycolide, without being not limited thereto.

The biodegradable polymer may include two or more types of polylactide-co-glycolide. The biodegradable polymer may include one type of polylactide-co-glycolide and one type of polylactide, without being limited to the above examples, and any polymer may be used without limitation as long as it may release CBD *in vivo* in a sustained manner for one month or more.

The microparticles may comprise CBD and the biodegradable polymer at a weight ratio of 1:3 to 1:10, 1:3.5 to 1:9.5, or 1:4 to 1:9. When the microparticles comprising CBD and the biodegradable polymer at a weight ratio within the above range are used as an injectable composition, they may be used as a sustained-release injectable composition that releases CBD in a sustained manner within an injectable dose range. That is, if the weight ratio of CBD to the biodegradable polymer is lower than the lower limit of the above range, the amount of the injectable composition will be increased to meet the total dose for administering CBD by injection, which causes a problem of exceeding the amount of the injectable composition that may be administered once to a person. In addition, if the weight ratio of CBD to the biodegradable polymer is higher than the upper limit of the above range, it will not be easy to produce microparticles in which CBD is uniformly distributed.

The inflammatory disease may be osteoarthritis. The sustained-release injectable composition may also be used for other inflammatory diseases, and may be applied without limitation to any disease for which CBD has been proven to be effective in treating or alleviating inflammatory diseases. However, the injectable composition may preferably be used for the treatment or alleviation of osteoarthritis.

The microparticles may release 35% to 80% of the total weight of CBD, as measured at 18 hours in a release test conducted under the following release test conditions:

### [Release test conditions]

A dissolution test solution is prepared by adding Tween 20 to 1 L of 10 mM PBS buffer (pH 7.4) to 0.05% (w/v), thus preparing a mixed solution, and adding sodium azide and sodium ascorbate to the mixed solution to 0.02% (w/v) and 0.0625% (w/v), respectively.

100 mL of the dissolution test solution is placed in a vial and the microparticles are added thereto in such an amount that the total amount of CBD is 15 mg. After completely sealing the vial, the test is conducted by shaking the vial at 120 rpm while maintaining 50°C. At 18 hours after the start of dissolution, the vial is taken out and left for 3 minutes. Then, 1 mL of the test solution is accurately taken using a syringe, and centrifuged at 3,000 rpm for 3 minutes, and the supernatant is used as a test solution to measure the release of CBD.

The CBD release test is conducted to confirm the sustained release of CBD using a release test solution. As described above, in order for microparticles containing CBD to release CBD in a sustained manner for one month or more, 35% to 80%, 40% to 78%, or 50% to 75% of the total weight of CBD should be released up to 18 hours in the release test. When CBD is released in an amount within the above range, the microparticles may exhibit the effect of releasing CBD in a sustained manner for one month or more. That is, if CBD is released in an amount lower than the lower limit of the above range, the effect of releasing CBD at the initial stage will be excessively small so that the effect of treating or alleviating inflammatory diseases by CBD may not be exhibited, and if CBD is released in an amount higher than the upper limit of the above range, the amount of CBD released will be excessively large so that the effect of releasing CBD in a sustained manner for one month or more may not be exhibited.

In addition, the microparticles of the present invention may further comprise butylated hydroxytoluene (BHT).

The CBD may be a compound represented by the following Formula:

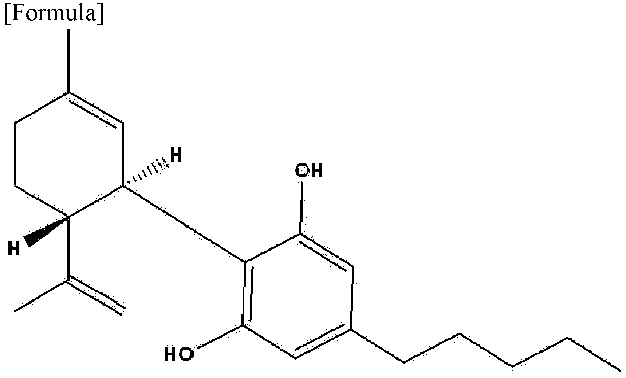

CBD, a compound represented by the above Formula, is a stable compound that is not oxidized upon contact with oxygen in the air. However, if CBD is formulated into microparticles comprising a biodegradable polymer as in the present invention, there is a problem in that oxidation of CBD occurs.

If CBD is oxidized as described above, the color may change to yellow as described above, and as the structure of the compound changes due to oxidation, a decrease in efficacy may occur.

The butylated hydroxytoluene (BHT) may be contained in the microparticles to prevent the oxidation of CBD. That is, the butylated hydroxytoluene (BHT) may prevent the oxidation of cannabidiol by being oxidized instead of CBD in the microparticles.

The microparticles of the present invention are spherical microparticles, as described below, and when the solvent is completely removed through the production process, cannabidiol, the sustained-release agent, and butylated hydroxytoluene (BHT) may be uniformly distributed therein.

At this time, the oxidation reaction of CBD occurs in an environment where the microparticles come into contact with oxygen, but the oxidation reaction of butylated hydroxytoluene (BHT) occurs preferentially to the oxidation reaction of CBD, so that the oxidation reaction of CBD may be prevented.

Although the oxidation reaction of CBD may be prevented by the above-mentioned action, this oxidation-preventing effect is not exhibited by an antioxidant other than butylated hydroxytoluene (BHT), and when the microparticles comprise butylated hydroxytoluene (BHT), butylated hydroxytoluene (BHT) may be oxidized preferentially to CBD, thereby exhibiting the effect of preventing the oxidation of CBD.

The butylated hydroxytoluene (BHT) may be comprised in an amount of 0.11 wt% to 9.9 wt%, 0.2 wt% to 9 wt%, 0.3 wt% to 8 wt%, or 0.5 wt% to 5 wt%, based on the total weight of cannabidiol and the biodegradable polymer. Within the above range, it is possible to produce spherical microparticles, it is possible to maintain an appropriate content range of CBD so that the microparticles may be used as a sustained-release formulation, and it is possible to increase the stability of CBD through the effect of preventing the oxidation of CBD by butylated hydroxytoluene (BHT).

A sustained-release injectable composition comprising cannabidiol according to another embodiment of the present invention may comprise: the microparticles containing cannabidiol; and a suspending solvent.

The injectable composition may comprise a suspending solvent, wherein the suspending solvent includes an isotonic agent, a suspending agent, and a solvent.

More specifically, the isotonic agent may be selected from the group consisting of D-mannitol, maltitol, sorbitol, lactitol, xylitol, sodium chloride, and mixtures thereof, and is preferably D-mannitol, without being limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, and is preferably sodium carboxymethylcellulose and polysorbate 80, without being limited to the above examples.

As the solvent, water for injection may be used, and any solvent that may be used as water for injection may be used without limitation.

A method for producing microparticles containing cannabidiol (CBD) according to another embodiment of the present invention may include steps of: preparing an oil phase solution by dissolving CBD and a biodegradable polymer in an organic solvent; preparing an aqueous phase solution by dissolving a surfactant in water; and mixing the oil phase solution and the aqueous phase solution to form an emulsion.

According to the production method, an emulsion may be formed using an oil phase solution prepared by dissolving CBD, a sustained-release agent, and an antioxidant in an organic solvent and an aqueous phase solution containing a surfactant.

As described below, according to the present invention, the emulsion is produced by a microfluidic method using a microchannel, without being limited to this production method, and any microparticle production method such as a solvent evaporation method or a membrane method may be applied.

The organic solvent may be one capable of completely dissolving CBD and the biodegradable polymer. Specifically, the organic solvent may be any one or more selected from the group consisting of chloroform, chloroethane, dichloroethane, dichloromethane, trichloroethane, methylene chloride, methanol, and mixtures thereof, and preferably may be selected from the group consisting of methylene chloride, methanol, and mixtures thereof. In addition to the above-listed organic solvents, any organic solvent may be used without limitation, as long as it is capable of completely dissolving CBD and the biodegradable polymer and may be easily selected by those skilled in the art.

The CBD and biodegradable polymer in the organic solvent may be contained at a weight ratio of 1:3 to 1:10, 1:3.5 to 1:9.5, or 1:4 to 1:9.

The biodegradable polymer in the oil phase solution is contained in an amount of 10 to 20 wt%, preferably 12 to 18 wt%, more preferably 15 wt%, without being to the above examples. If the biodegradable polymer is contained in an amount smaller than the lower limit of the above range, it is impossible to prepare an emulsion, and if biodegradable polymer is contained in an amount larger than the upper limit of the above range, there is a problem in that the viscosity of the oil phase solution is excessively high, making it not easy to prepare an emulsion.

The biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof, and is preferably polylactide-co-glycolide (PLGA) and/or polylactide (PLA), without being limited to the above examples.

The oil phase solution may further contain butylated hydroxytoluene (BHT). As described above, the butylated hydroxytoluene (BHT) may be contained in the oil phase solution and may be contained in the microparticles, thereby serving to prevent the oxidation of CBD. The butylated hydroxytoluene (BHT) may be contained in an amount of 0.11 wt% to 9.9 wt%, 0.2 wt% to 9 wt%, 0.3 wt% to 8 wt%, or 0.5 wt% to 5 wt%, based on the total weight of cannabidiol and the biodegradable polymer.

The aqueous phase solution may contain the surfactant in an amount of 0.1 to 1.0 wt%, 0.2 to 0.5 wt%, or 0.25 wt%, with the remainder being water.

The surfactant may be any one or more selected from the group consisting of methylcellulose, polyvinylpyrrolidone, lecithin, gelatin, polyvinyl alcohol, sorbitan monooleate (e.g., Span^{™} 80, etc.), polyoxyethylene sorbitan fatty acid ester (e.g., Tween^{™} 80, etc.), polyoxyethylene castor oil derivatives, sodium lauryl sulfate, sodium stearate, ester amines, linear diamines, patty amines, and mixtures thereof, and preferably may be polyvinyl alcohol, without being limited to the above examples, and any surfactant that may be prepared into a perfectly spherical emulsion may be used.

After the oil phase solution and the aqueous phase solution are prepared as described above, the method of preparing an emulsion using the same is not limited.

However, in the present invention, a method of producing microparticles using a microfluidic method will be described.

A microchip for using the microfluidic method may be formed on a wafer or a glass substrate. Microchannels are formed in the microchip. More specifically, the microchannels include a channel through which the oil phase solution flows, a channel through which the aqueous phase solution flows, and a transport channel. The channel through which the oil phase solution flows and the channel through which the aqueous phase solution flows are formed to meet each other at one point, and one end of the transport channel may be connected to a portion where the two channels are joined.

Injection portions for injecting the oil phase solution and the aqueous phase solution are connected to the channel through which the oil phase solution flows and the channel through which the aqueous phase solution flows, respectively, and a recovery portion for recovering a solution containing an emulsion may be connected to one end of the transport channel.

The microchannels may be formed on a material selected from the group consisting of a glass substrate, a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited to the above examples.

As an example, aluminum is deposited on a silicon wafer using an e-beam evaporator, and photoresist is patterned on the aluminum using a photolithography technique. Thereafter, the aluminum is etched using the photoresist as a mask, the photoresist is removed, the silicon wafer is etched by deep ion reactive etching (DRIE) using the aluminum as a mask, the aluminum is removed, and then glass is anodically bonded onto the wafer and hermetically sealed, thereby fabricating the microchannels.

The microchannels have an average diameter of 60 to 150 µm, preferably 80 to 100 µm, without being limited to the above example. If microchannels having an average diameter smaller than the lower limit of the above range are used, an emulsion having an excessively small diameter may be produced, which may affect the release and *in vivo* absorption of the effective drug.

In addition, if the microchannels have an average diameter larger than the upper limit of the above range, the average size of the produced microparticles will exceed 120 µm, and foreign body sensation and pain may increase when the microparticles are administered by injection. As the diameter of the microchannels increases, the particle size distribution of the produced microparticles increases, making it difficult to produce microparticles with a uniform particle size.

In addition, the average diameter of the microchannels is closely related not only to the average diameter of the particles, but also to the ratio of the flow rates (µl/min) of the oil phase solution and the aqueous phase solution.

In addition, the cross-sectional width (w) and the cross-sectional height (d) of the microchannel are closely related to the average diameter (d') of the microparticles being produced. The cross-sectional width (w) of the microchannel is in a ratio range of 0.7 to 1.3 with respect to the average diameter (d') of the microparticles, and the cross-sectional height (d) of the microchannel is in a ratio range of 0.7 to 1.3 with respect to the average diameter (d') of the microparticles.

That is, when the average diameter (d') of the microparticles to be produced is determined, the lengths of the cross-sectional width (w) and cross-sectional height (d) of the microchannel should be set in a ratio range of 0.7 to 2.3 with respect to d' so that microparticles having a desired size may be produced.

In order to produce microparticles using the above-described microchip, the oil phase solution may be injected into the channel through which the oil phase solution flows, and the aqueous phase solution may be injected into the channel through which the aqueous phase solution flows, thereby forming an emulsion at the portion where the two channels are joined.

When the oil phase solution and the aqueous phase solution are injected into the respective microchannels, the ratio of the flow rates of the oil phase solution and the aqueous phase solution may be 1:10 to 1:50, 1:15 to 1:40, 1:15 to 1:30, or 1:15 to 1:25. By controlling the ratio of the flow rates of the oil phase solution and the aqueous phase solution as described above, it is possible to produce microparticles having a uniform diameter.

The emulsion formed in the portion where the two channels are joined may be collected using the previously prepared aqueous phase solution, i.e., a mixed solution of the surfactant and water. The prepared aqueous phase solution may be used to form an emulsion by being injected into the microchannel, and may also be used to prevent the aggregation of emulsion particles by being placed into a bath.

Residual organic solvent may be removed from the emulsion collected in the bath. The step of removing the residual organic solvent may be performed by stirring the emulsion at a predetermined temperature and a predetermined stirring speed to evaporate and remove the residual organic solvent present in the emulsion. Here, the stirring is performed under the following conditions: a first stirring step at 15 to 20°C for 50 to 70 minutes; a second stirring step at 20 to 40°C for 50 to 70 minutes; and a third stirring step at 40 to 60°C for 1 to 3 hours.

The stirring speed is the same for all of the first to third stirring steps, and the stirring speed may be 300 to 500 rpm, or 400 rpm.

As described above, the stirring temperature is gradually increased as the stirring process progresses. By increasing the temperature stepwise, it is possible to control the evaporation rate of the organic solvent present in the emulsion.

The temperature at which the oil phase solution and the aqueous phase solution flow through the microchannels is also 5 to 20°C, preferably 10°C. That is, after the solutions flow along the microchannels and intersect each other to produce an emulsion, the collected emulsion is maintained at a constant low temperature of 5 to 20°C until it is stirred in the first stirring step. Only when the emulsion preparation process is maintained at a low temperature, it is possible to produce and maintain spherical particles. That is, if a low-temperature condition is not used, a problem arises in that it is difficult to produce particles having a uniform spherical shape.

Thereafter, in the second stirring step, the temperature is increased gradually and the stirring time is increased so that the organic solvent present in the emulsion may be moved slowly to the surface and evaporated from the surface, thereby minimizing the effect of the evaporation of the organic solvent on the appearance of the emulsion. That is, if the organic solvent is rapidly evaporated, a problem may arise in that the surfaces of the finally produced microparticles are not smooth and pores are formed, due to the evaporation of the organic solvent. In order to prevent this problem, the evaporation rate of the organic solvent may be controlled by increasing the temperature gradually as described above and also increasing the stirring process time, and due to this control of the evaporation rate of the organic solvent, it is possible to control the surface appearance of the produced microparticles.

In the third stirring process, after the organic solvent in the emulsion is extracted with the external aqueous phase, the external aqueous phase is stirred at an increased temperature near the boiling point of the organic solvent, thereby removing the saturated organic solvent from the aqueous phase, thus facilitating the removal of residual organic solvent from the emulsion.

Lastly, a step of washing and drying the microparticles from which residual organic solvent has been removed is performed. In this step, the microparticles from which the organic solvent on the surface has been completely removed by stirring are washed several times with sterile filtered purified water to remove the surfactant remaining in the microparticles, and then freeze-dried.

The final microparticles are formed in a form in which CBD is uniformly distributed in the microparticles made of the spherical biodegradable polymer, and may comprise CBD and the biodegradable polymer at a weight ratio of 1:3 to 1:10.

In addition, as described above, the finally produced microparticles have butylated hydroxytoluene (BHT) uniformly distributed therein in addition to CBD, so that when they come into contact with oxygen in the air, butylated hydroxytoluene (BHT) may be preferentially oxidized, thereby preventing the oxidation of CBD.

The weight ratio between CBD and the biodegradable polymer contained in the microparticles is the same as the weight ratio in the oil phase solution used to prepare the emulsion. Specifically, as the solutions pass through the microchannels to form the emulsion and the organic solvent is completely removed from the emulsion, the produced microparticles may comprise CBD and the biodegradable polymers at the same weight ratio as the weight ratio in the oil phase solution.

The microparticles may have an average diameter of 30 to 70 µm, 30 to 65 µm, or 30 to 60 µm. In addition, the standard deviation for the average diameter may be 1 to 30 µm, 1 to 20 µm, 1 to 10 µm, or 1 to 7 µm. It can be confirmed that it is possible to produce uniform particles within the above diameter range. When the uniform particles are used as a sustained-release injectable composition, they may reduce foreign body sensation, increase the convenience of administration, prevent initial over-release upon *in vivo* injection, and exhibit the effect of releasing CBD in a sustained manner.

### Production Example

### Production of Microparticles

### 1) Preparation of Oil Phase Solution

An oil phase solution was prepared by dissolving cannabidiol, a biodegradable polymer consisting of at least one of a lactide-glycolide copolymer and a lactide copolymer, and butylated hydroxytoluene (BHT) in methylene chloride. Here, the content of the biodegradable polymer in the oil phase solution was 15 wt%, and the weight ratio between cannabidiol and the biodegradable polymer was 1:3 to 1:10.

### 2) Preparation of Aqueous Phase Solution

An aqueous phase solution was prepared by dissolving polyvinyl alcohol in water. The content of polyvinyl alcohol in the aqueous phase solution was 0.25 wt%.

### 3) Production of Microparticles

The oil phase solution and the aqueous phase solution were injected into microchannels formed on a silicon wafer, thereby producing microparticles. Here, the ratio of the flow rate of the oil phase solution to the flow rate of the aqueous phase solution was 1:20, and the temperature was maintained at 10.0°C. The produced microparticles were collected in a bath containing the water phase solution, and stirred at a speed of 400 rpm at 10.0°C for 1 hour, or at 30.0°C for 1 hour, or at 45.0°C for 2 hours.

### 4) Washing and Collection

After completion of the stirring, the microparticles were washed with sterile filtered purified water, freeze-dried, and then collected in powder form.

Microspheres were produced using the components and contents shown in Table 1 below according to the method described above.

**[Table 1]**

| Purpose of mixing | Component name | Comp. Example 1 | Comp. Example 2 | Example 1 | Example 2 | Example 3 | Example 4 | Example 5 |
|---|---|---|---|---|---|---|---|---|
| | | Quantity (mg) | | | | | | |
| Active ingredient | Cannabidiol | 100.00 | | | | | | |
| Sustained-release agent | Lactide-glycolide copolymer (50:50) | - | - | 400.00 | - | - | 267.00 | - |
| | Lactide-glycolide copolymer (75:25) | 300.00 | 1,000.00 | - | 400.00 | 900.00 | 133.00 | 267.00 |
| | Lactide copolymer | - | - | - | - | - | - | 133.00 |
| Antioxidant | Butylated hydroxytoluene | 1.00 | | | | | | |
| Organic solvent | Methylene chloride | 2,000.00 | 6,666.67 | 2,666.67 | 2,666.67 | 6,000.00 | 2,666.67 | 2,666.67 |
| Total mass (mg) | | 401.00 | 1,101.00 | 501.00 | 501.00 | 1,001.00 | 501.00 | 501.00 |

### Experimental Example 1

### CBD Release Experiment

### 1) Preparation of Standard Solution

About 15 mg of cannabidiol standard was taken and placed in a 100-mL flask. About 50 mL of diluted solution (acetonitrile) was added thereto and the mixture was sonicated for 10 minutes. After cooling sufficiently, the test solution was added up to the marked line, filtered through a 0.45 µm filter (water-soluble PTFE), and used as a standard solution (concentration: 0.15 mg/mL).

### 2) Preparation of Dissolution Test Solution

A dissolution test solution was prepared by adding Tween 20 to 1 L of 10 mM PBS buffer (pH 7.4) to 0.05% (w/v), and then adding sodium azide and sodium ascorbate to the mixture to 0.02% (w/v) and 0.0625% (w/v), respectively.

### 3) Preparation of Test Solution

Each sample was precisely weighed to be 15 mg as the main ingredient, and placed in a vial, and 100 mL of the dissolution test solution was accurately added thereto, sealed, and then tested by shaking at a speed of 120 rpm and at a constant temperature of around 50°C. The vial was placed horizontally. The vial containing the sample was taken out at 2, 18, and 72 hours from the start of dissolution and left for 3 minutes so that the sample did settle down in the test solution. Next, 1 mL of the test solution was accurately taken using a syringe and centrifuged at 3,000 rpm for 3 minutes, and the supernatant was used as a test solution.

### 4) HPLC conditions

- Detector: UV (220 nm)
- Column: ODS column (4.6*150mm, 5 µm)
- Flow rate: 1.0 mL/min
- Mobile phase: methanol: purified water = 85:15 (isocratic method)
- Calculation formula: Main peak area of the test solution*amount of standard taken*purity of standard (%)*dilution ratio/peak area of main component of standard solution*equivalent amount (mg) of cannabidiol in test solution

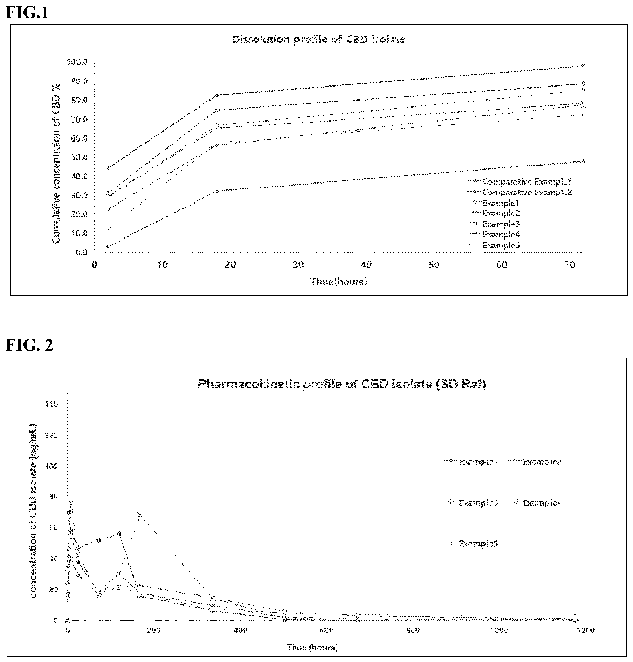

The results of conducting the CBD release experiment under the above-described experimental conditions are shown in FIG. 1 and Table 2 below.

**[Table 2]**

| | 2 hours | 18 hours | 72 hours |
|---|---|---|---|
| Comparative Example 1 | 44.5±0.3 | 82.6±2.4 | 97.9±0.2 |
| Comparative Example 2 | 3.0±3.9 | 32.2±0.1 | 47.9±0.1 |
| Example 1 | 31.2±6.5 | 74.8±0.1 | 88.6±0.5 |
| Example 2 | 29.6±0.4 | 65.2±3.8 | 78.2±0.7 |
| Example 3 | 22.9±10.6 | 56.4±2.5 | 77.4±1.8 |
| Example 4 | 28.9±0.6 | 66.7±1.2 | 85.0±0.3 |
| Example 5 | 12.1±8.2 | 57.8±1.0 | 72.3±0.2 |

Referring to the above experimental results, it can be confirmed that Comparative Example 1 showed CBD release amounts of 44.5% at 2 hours, 82.6% at 18 hours, and 97.9% (almost completely released) at 72 hours. Comparative Example 1 has a problem in that it cannot exhibit the effect of releasing CBD in a sustained manner for a long period of time, as the initial release amount of CBD is large.

In addition, Comparative Example 2 showed a CBD release amount of 47.9% at 72 hours, indicating that it can exhibit the effect of releasing CBD in a sustained manner for a long period of time. However, it showed a CBD release amount of only 3% at 2 hours, indicating that the initial CBD release amount was excessively small. Thus, it has a problem in that the effect of CBD on the treatment and alleviation of inflammatory diseases is insufficient.

On the other hand, Examples 1 to 5 could show an appropriate level of CBD release at a time point of 2 hours, and did not show complete release of CBD even at 72 hours, suggesting that they exhibited a drug release effect in a sustained manner for a long period of time.

### Experimental Example 2

### Rat Pharmacokinetic Test

The experimental conditions for conducting the rat pharmacokinetic test are as follows:

**[Table 3]**

| Administration route | Subcutaneous (SC) injection |
|---|---|
| Administration frequency and duration | Single administration |
| Administration site and dose (mg/kg) | Back skin tissue; CBD solution: 300 µg/head, IVL5005: 9 mg/head |
| Method of administration | SC: Inject subcutaneously into the skin tissue of the back using a 1-ml disposable syringe. |

CBD isolate solution was used as a control. As experimental animals, 6-week-old male SD rats were purchased from Coretech, acclimated for a week, and then tested. The experimental diet was a general feed for experimental animals (Orient), and the rats were raised with free access to water and feed.

### General Symptoms

During the experimental period, changes in general symptoms were observed twice (morning and afternoon) a day for all animals, and body weight was measured upon introduction and group separation.

### Blood Sampling

At a total of 14 time points, including time 0 (before SC administration of the test substance to the acclimated rats) and 4 hr, 8 hr, 24 hr, 3 d, 5 d, 1 w, 2 w, 4 w, 6 w, 8 w, and 12 w after SC administration of the test substance, 500 to 1,000 µL of blood was sampled from the jugular vein at each time point, and then immediately, the blood was centrifuged at 1,000 g for 10 minutes to separate the serum, which was then frozen and stored at -70°C.

The results of measuring pK for the microparticles of Examples 1 to 5 in the above experiment are shown in Table 4 below and FIG. 2.

**[Table 4]**

| Time | Control | Example 1 | Example 2 | Example 3 | Example 4 | Example 5 |
|---|---|---|---|---|---|---|
| 1 d | 3.94 | 46.793 | 37.420 | 29.238 | 43.827 | 42.296 |
| 1 w | 0.361 | 15.344 | 17.496 | 22.324 | 67.629 | 17.295 |
| 2 w | 0 | 6.005 | 9.634 | 14.640 | 14.133 | 7.107 |
| 4 w | 0 | 0.036 | 0.595 | 2.539 | 0.782 | 3.771 |
| 7 w | 0 | 0.000 | 0.364 | 0.761 | 0.000 | 3.401 |

(unit: µg/mL) Referring to the above experimental results, it can be confirmed that Examples 1 to 5 of the present invention maintained the blood CBD concentration for at least 1 month. That is, it was confirmed that Example 1 showed 0.036 µg/mL at 4 weeks, and Example 4 showed 0.782 µg/mL at 4 weeks.

In addition, it was confirmed that Examples 2 to 5 exhibited the effect of releasing CBD in a sustained manner as they showed the blood CBD concentration even at 7 weeks.

### Experimental Example 3

### Trial for Treatment of Osteoarthritis (OA)

After acclimatization, rats were divided into groups, and the area around the left lower limb knee joint of each rate was shaved clean. Then, MIA (monosodium iodoacetate, Sigma, USA), an osteoarthritis-inducing substance, was prepared at a concentration of 60 mg/mL in 0.9% sterile saline and 50 µL was administered intra-articularly to each rat using a BD Ultra-Fine^{™} II Insulin Syringe. The right knee joint was administered only the same volume of 0.9% sterile saline. Two weeks after MIA administration, the test substances shown in Table 5 below were administered intra-articularly, and the therapeutic efficacy thereof was evaluated.

**[Table 5]**

| Group | N | Route | Dose level* | Dose volume | Regimen | Necropsy |
|---|---|---|---|---|---|---|
| Normal control | 10 | IA (Intra-articular) | - | - | Saline -> Vehicle | D28 |
| Positive control | 10 | | - | - | MIA -> Vehicle | |
| CBD solution | 10 | | 300 ug/joint | 30 ul/joint | MIA -> CBD sol. | |
| Example 2 | 10 | | 2 mg/joint | 30 ul/joint | MIA -> IVL5005 | |
| Example 4 | 10 | | 2 mg/joint | 30 ul/joint | | |
| Example 3 | 10 | | 1 mg/joint | 30 ul/joint | | |

### Method of Administration

CBD solution and Examples 2 to 4 were each administered to the left knee joint cavity 14 days after MIA administration, and only the same amount of 0.9% sterile saline was administered to the right knee joint. The normal control and positive control groups were also administered the same amount of 0.9% saline.

### Dose Level Selection

- CBD solution: The effective concentration of 300 µg/joint in the MIA-OA model was selected based on existing literature.
- Examples 2 and 3: Maximum feasible dose level (1 to 2 mg/joint) considering the microsphere volume and rat intra-articular dose volume (30 µl)

### Weight Measurement Results

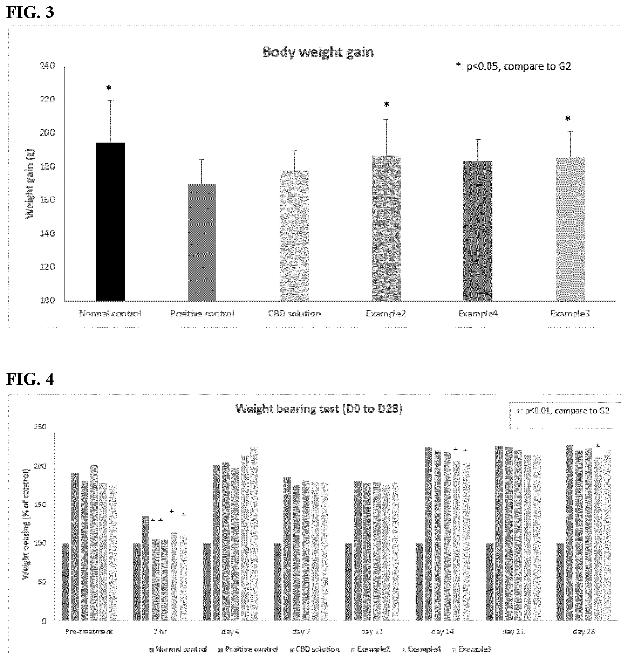

The results of body weight measurement during the test period are as shown in FIG. 3. The body weight gain was the lowest in the positive control group, and the body weight gain in the CBD solution- and Examples 2 to 4-administerd groups tended to increase compared to that in the positive control group. The body weight gain in the Example 3 and Example 4-administered groups statistically significantly increased compared to that in the positive control group. When OA was induced, a decrease in feed intake due to pain and walking difficulties was generally observed, and for this reason, the body weight tended to decrease. However, when CBD was administered, it was confirmed that the body weight gain increased compared to that in the positive control as feed intake activity was improved due to a decrease in pain. This tendency was particularly significant in Examples 3 and 4.

### Measurement of Hind Limb Weight Bearing

Hind limb weight bearing was measured before MIA administration (day 0) and 6 and 13 days after MIA administration (before test drug administration), and was measured for the left and right paws 2 hours, 4 hours, 3 days, 6 days, 10 days, 13 days, 20 days, and 27 days after test drug administration using an incapacitance tester (Ugo Basile, Italy). The weight of each paw (g) was measured in a state in which the rat's abdomen did not touch the sensor of the device. The measurement results were analyzed as the ratio (%) of the weights measured on the left and right paws (left/right).

The weights (g) of both hind limbs were measured on each measurement day. When arthritis was induced, the weight bearing on the arthritis-induced side (left) was reduced. The weight-bearing value was calculated by the following equation: Weight-bearing value = normal hind limb weight (right)/arthritis-induced hind limb weight (left) Relative pain ratio (%) = (average weight bearing value of each group/average weight bearing value of normal group) X 100

The test results are shown in FIG. 4.

It was confirmed that the positive control group had induced arthritis with a significant change in weight bearing compared to the normal control group. At 2 hours after administration, the CBD solution- and Example 2 to 4-administered groups showed significant improvement in weight bearing compared to the positive control (PC) group.

The CBD solution-administered group showed no difference compared to the PC group at 2 hours after administration. The weight bearing in the Example 4-administered group tended to improve compared to that in the PC group after 14 days after administration, and was statistically significantly improved compared to that in the control group on days 14 and 28. It was confirmed that the pain in the Example 3-administered group tended to decrease compared to that in the PC group on days 14 and 28 after administration and was significantly reduced on day 14.

### Paw Withdrawal Threshold Test

The paw withdrawal response was measured before MIA administration (day 0) and 7 days and 14 days after MIA administration (before test drug administration), and the paw withdrawal response was evaluated after applying mechanical stimulation using an electronic von-Frey aesthesiometer (IITC Life Science, USA) 2 hours, 4 hours, 4 days, 7 days, 11 days, 14 days, 21 days, and 28 days after test drug administration. To measure the paw withdrawal response, the rat was placed in a transparent acrylic container on a wire mesh and allowed to adapt to the new environment for about 20 minutes. In the measurement method, an electronic von-Frey filament was applied perpendicular to the sole of the paw, the threshold (g force) value at which a rapid withdrawal response occurred was measured. The measurement results were analyzed as the ratio of g forces measured on the left and right paws (left/right).

Paw withdrawal threshold ratio = Left threshold (arthritis-induced side)/Right threshold (normal side). When arthritis is alleviated, the left-right ratio becomes closer to 1 (ratio 1)

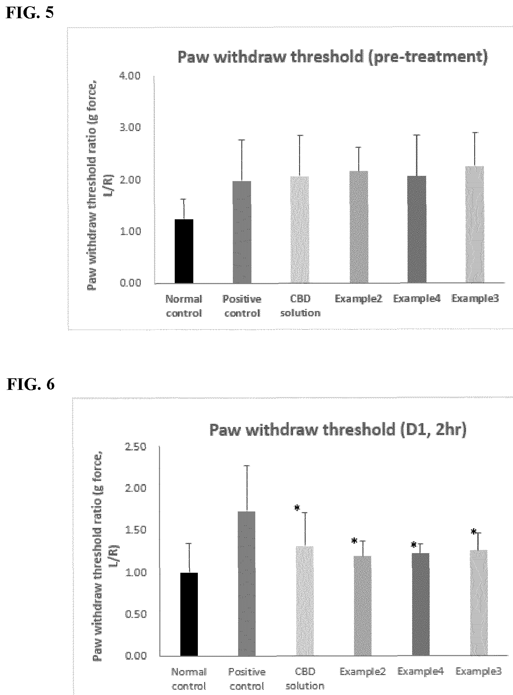

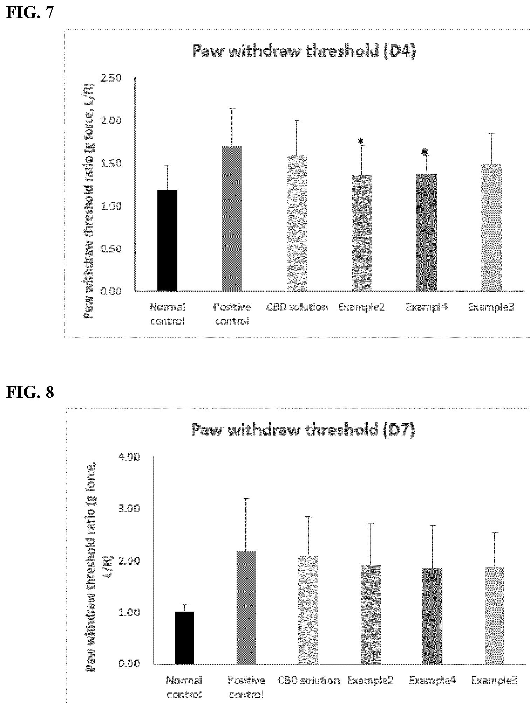

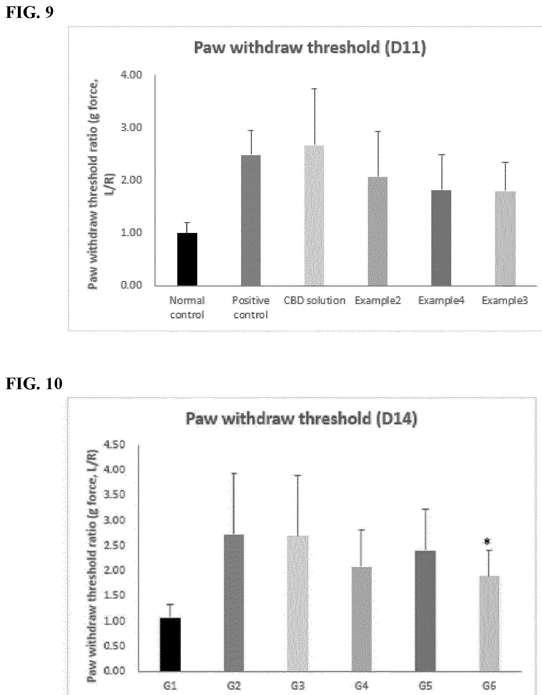

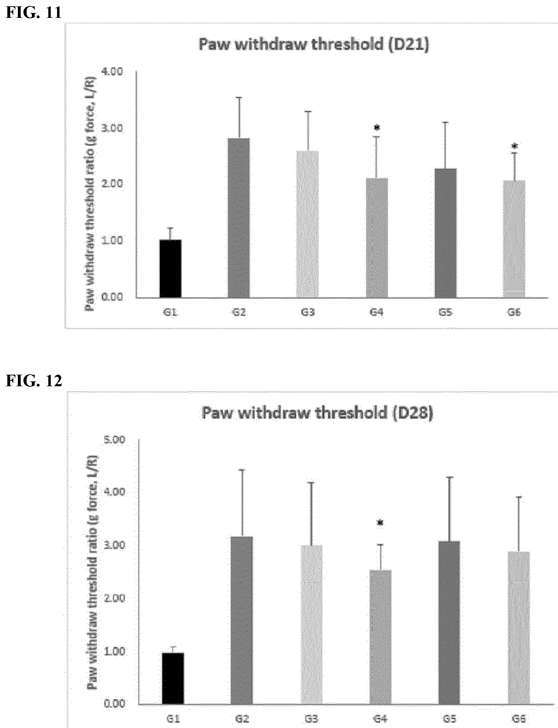

The test results are shown in FIGS. 5 to 12.

The positive control (PC) group showed a significant increase in paw withdrawal threshold ratio compared to the normal control group. At 2 hours after administration, the CBD solution- and Example 2 to 4-adminstered groups all showed a significant improvement in the response compared to the PC group. Specifically, the paw withdrawal threshold ratio in the CBD solution-administered group tended to decrease compared to that in the PC group at 2 hours after administration, and thereafter, there was no difference compared to the control group. The paw withdrawal threshold ratio in the Example 2-administered group tended to decrease continuously even after day 4 after administration, and was statistically significantly reduced on days 4, 21 and 28. The paw withdrawal threshold ratio in the Example 4-administered group tended to decrease continuously even after day 4 after administration, and was statistically significantly reduced on days 4 and 11. Finally, the paw withdrawal threshold ratio in the Example 3-administered group tended to decrease continuously even after day 4 after administration, and was statistically significantly reduced on days 11, 14 and 21.

Referring to the results of the hind limb weight-bearing test and the paw withdrawal threshold test, it could be confirmed that the CBD solution-administered group showed an effect compared to the PC group in the early stage of administration, but thereafter, that there was no difference from the PC group. On the other hand, it was confirmed through the tests that Examples 2 to 4 exhibited a long-term, sustained therapeutic effect compared to the PC group, indicating that they not only exhibited the effect of treating and alleviating osteoarthritis in the osteoarthritis model, but they also exhibited a long-term, sustained therapeutic effect.

### Experimental Example 4

### Evaluation of Stability

Microparticles were produced using the same composition as that of Example 1 in the same manner as in Example 1, except that BHT was not included.

About 100 mg of the microparticles were weighed into a 5-mL glass vial which was then stored in a capped or uncapped (close/open) stability chamber. Stability evaluation was performed under long-term conditions (temperature: 25±2°C, relative humidity: 60±5%), and daily appearance changes were observed to check yellowing. Meanwhile, the initial sample and the sample on day 6 after storage were tested for their encapsulation rate to check for any changes in content.

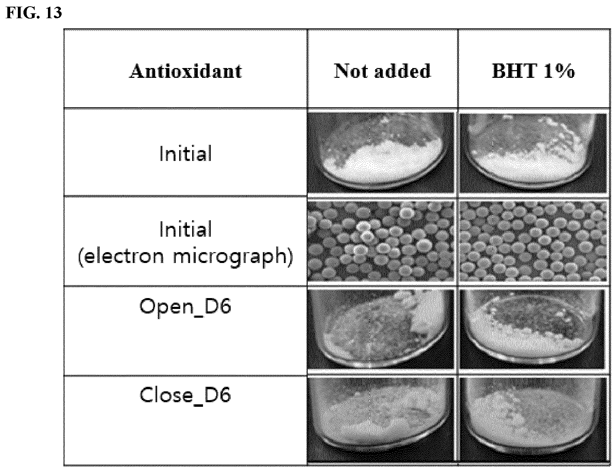

The experimental results are shown in FIG. 13.

It was confirmed that there was no change in color immediately after producing the microparticles. In addition, as a result of checking the presence of yellowing on day 6 after storage, it was confirmed that the color changed to yellow in the case in which BHT was not included, regardless of whether the glass vial was capped.

On the other hand, it was confirmed that, in the case in which BHT was included in an amount of 1 wt% as in Example 1, the color did not change, indicating excellent stability.

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

### Industrial Applicability

The present invention relates to a sustained-release injectable composition for treating or preventing inflammatory diseases and a method for preparing the same.

## Claims

1. A sustained-release injectable composition for treating or preventing an inflammatory disease, comprising microparticles that release cannabidiol (CBD) in a sustained manner for one month or more,
wherein the microparticles comprise cannabidiol and a biodegradable polymer.

2. The sustained-release injectable composition of claim 1, wherein the microparticles comprise cannabidiol and the biodegradable polymer at a weight ratio of 1:3 to 1:10.

3. The sustained-release injectable composition of claim 1, wherein the biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

4. The sustained-release injectable composition of claim 3, wherein the polylactide-co-glycolide has a monomer ratio of lactide: glycolide of 40: 60 to 90: 10.

5. The sustained-release injectable composition of claim 1, wherein the inflammatory disease is osteoarthritis.

6. The sustained-release injectable composition of claim 1, wherein the microparticles release 35% to 80% of the total weight of CBD, as measured at 18 hours in a release test conducted under the following release test conditions:
[Release test conditions]
A dissolution test solution is prepared by adding Tween 20 to 1 L of 10 mM PBS buffer (pH 7.4) to 0.05% (w/v), thus preparing a mixed solution, and adding sodium azide and sodium ascorbate to the mixed solution to 0.02% (w/v) and 0.0625% (w/v), respectively.
100 mL of the dissolution test solution is placed in a vial and the microparticles are added thereto in such an amount that the total amount of CBD is 15 mg. After completely sealing the vial, the test is conducted by shaking the vial at 120 rpm while maintaining 50°C. At 18 hours after the start of dissolution, the vial is taken out and left for 3 minutes. Then, 1 mL of the test solution is accurately taken using a syringe, and centrifuged at 3,000 rpm for 3 minutes, and the supernatant is used as a test solution to measure the release of CBD.

7. A method for preparing a sustained-release injectable composition for treating or preventing an inflammatory disease, comprising steps of:
preparing an oil phase solution by dissolving cannabidiol (CBD) and a biodegradable polymer in an organic solvent;
preparing an aqueous phase solution by dissolving a surfactant in water; and
mixing the oil phase solution and the aqueous phase solution to form an emulsion.

8. The method of claim 7, further comprising steps of:
collecting the formed emulsion in the aqueous phase solution to remove residual solvent;
washing and freeze-drying the emulsion from which the residual solvent has been removed, thereby producing microparticles; and
mixing the freeze-dried microparticles with water for injection.

9. The method of claim 7, wherein the oil phase solution and the aqueous phase solution are injected into the respective microchannels and allowed to flow therethrough, and the emulsion containing cannabidiol and the biodegradable polymer is formed at a point where the flow of the oil phase solution and the flow of the aqueous phase solution intersect each other.

10. The method of claim 7, wherein the oil phase solution further contains butylated hydroxytoluene (BHT).
